# EUROPÄISCHE PATENTSCHRIFT

(11) **EP 1 981 458 B1**
(45) Veröffentlichungstag und Bekanntmachung des Hinweises auf die Patenterteilung: **30.11.2016**
(21) Anmeldenummer: 07703270.4
(22) Anmeldetag: 05.02.2007
(51) Int. Cl.: A61F 13/02

(54) **FOLIENVERBAND MIT VERBESSERTER APPLIKATIONSHILFE**
FILM STRUCTURE WITH IMPROVED APPLICATION ASSISTANCE
ASSEMBLAGE DE FILMS PRÉSENTANT UN AUXILIAIRE D'APPLICATION AMÉLIORÉ

(30) Priorität: 07.02.2006 DE 102006005348
(43) Veröffentlichungstag der Anmeldung: 22.10.2008
(73) Patentinhaber: Paul Hartmann AG, 89522 Heidenheim (DE)
(72) Erfinder: ECKSTEIN, Axel, 89522 Heidenheim (DE)
(86) Internationale Anmeldenummer: PCT/EP2007/000960
(87) Internationale Veröffentlichungsnummer: WO 2007/090592

(56) Entgegenhaltungen:
- EP-A- 0 985 391
- EP-A1- 0 081 990
- EP-A1- 0 630 628
- EP-A2- 0 341 045
- EP-A2- 1 093 780
- EP-B1- 0 690 706
- EP-B1- 0 951 263
- WO-A-2006/092244
- WO-A-2006/092248
- WO-A-2006/133794
- US-A1- 2002 107 466

## Beschreibung

Die vorliegende Erfindung betrifft einen Folienverband mit einem Polymerfilm und einem Applikationssystem zur leichteren Handhabung des Folienverbandes.

Applikationshilfen für Pflaster oder Wundauflagen sind seit geraumer Zeit bekannt. Insbesondere kommen diese Applikationshilfen bei Film- oder Folienverbänden zum Einsatz. Folienverbände sind dünne, meist transparente, semipermeable Filme oder Folien aus Polymermaterialien. Die Semipermeabilität der Folien verhindert das Eindringen von Bakterien oder Nässe und gestattet dabei einen ausreichenden Sauerstoff- und Wasserdampfaustausch zwischen der abzudeckenden Haut und der äußeren Umgebung des Folienverbandes. Diese Film- oder Folienverbände kommen in vielfältiger Weise zum Einsatz zum Beispiel als Inzisionsfolie zur keimfreien Abdeckung von Operationsstellen, als separate wasserdichte Abdeckung von Exsudat aufsaugenden Wundauflagen sowie zur Fixierung von Kanülen oder Kathetern. Aufgrund der geringen Dicken dieser Filme oder Folien und der damit verbundenen Instabilität, werden diese Folienverbände mit den unterschiedlichsten Applikationshilfen ausgestattet. Die meisten dieser Applikationshilfen bedienen sich einer zusätzlichen Stützschicht, die während oder nach der Applikation des Folienverbandes entfernt werden.

Filmwundauflagen sind seit geraumer Zeit auch in der Patentliteratur bekannt. So beschreibt die EP 81 990 B1 eine adhäsive Wundauflage, die aus einem dünnen Polymerfilm besteht. Dieser Polymerfilm ist auf einer ersten Seite mit einem auf der Haut klebenden Haftklebstoff beschichtet, der seinerseits mit einer Ablöseschicht abgedeckt ist. Auf der anderen, in Gebrauch körperabgewandten Seite weist der Polymerfilm zur leichteren Handhabung zudem eine leicht zu entfernende Stützschicht aus einem Fasermaterial zum Beispiel ein undurchsichtiges Nonwoven auf. Diese Stützschicht weist dieselbe Größe wie der Polymerfilm auf.

Mit EP 690 706 B1 wird ein klebender Wundverband beschrieben, der zur leichteren Applikation eines Polymerfilms, der von dem Wundverband umfasst ist, eine Trägerschicht aufweist. Diese Trägerschicht deckt vollflächig den Polymerfilm ab und kann in zwei Schritten von dem Polymerfilm entfernt werden. Hierzu wird im ersten Schritt ein Mittelteil aus der Trägerschicht entfernt, worauf im zweiten Schritt ein Rahmenteil entfernt wird. Nachteilig ist, dass die Trägerschicht dieses Wundverbandes nur sehr schwer vom Benutzer ergriffen werden kann.

Des Weiteren beschreibt die EP 951 263 B1 einen klebenden Folienverband, dessen klebende Seite mit einer mindestens zweiteiligen abziehbaren Schutzschicht abgedeckt ist und dessen nicht klebende zweite Seite vollflächig mit einer einteiligen Stützschicht ausgestattet ist. Bei diesem Folienverband ist die Stützschicht mit der Schutzschicht an zwei gegenüberliegenden Seiten scharnierartig verbunden, so dass mit dem Entfernen der Schutzschicht gleichzeitig die Stützschicht entfernt werden kann.

Die EP 985 391 A1 beschreibt ein Verbandmaterial auf Folienbasis, das im Randbereich der Folie einen nicht klebenden Anfasser aufweist. Die nicht klebende Seite der Folie ist mit einer einteiligen Stützschicht versehen, die die gleiche Größe wie die Folie aufweist und mindestens eine Griffleiste aufweist. Durch Ziehen an dem Anfasser in Verklebungsrichtung kann die applizierte Folie wieder schmerzfrei entfernt werden.

Durch die europäische Patentschrift EP 630 628 B1 ist ein Filmverband bekannt, der zur leichteren Applikation eine zweigeteilte Stützfolie umfasst. Diese Stützfolie ist größer als der zu applizierende Film und bedeckt diesen vollflächig. Zum Entfernen der Stützfolie ist auf der Stützfolie ein weiterer adhäsiv ausgerüsteter Abziehstreifen angebracht, der über der Schnittlinie der Stützfolie angeordnet ist und zum Ergreifen zwei nicht klebende Randbereiche aufweist, die als Griffleiste dienen. Mit Hilfe dieses weiteren Abziehstreifens kann lediglich ein Teil der Stützfolie entfernt werden, worauf der zweite Teil der Stützfolie auf dem Polymerfilm verbleibt.

Darüber hinaus ist mit der europäischen Patentschrift EP 341 045 B1 ein klebender, transparenter oder transluzenter Folienverband beschrieben, der eine transparente Wundauflage und eine transparente, mit Bezugzeichen versehene Folie umfasst. Diese Bezugszeichen können zum Beobachten der Wunde verwendet werden.

Diese Schutzrechte zeigen insgesamt verschiedene alternative Lösungen zu Film- oder Folienverbänden mit verschiedenen Applikationssystemen auf. Teilweise werden die mit diesen Schutzrechten als Lösung vorgeschlagenen Folienverbände als zu kompliziert im Aufbau oder als unkomfortabel in der Anwendung angesehen.

Aufgabe der vorliegenden Erfindung ist es, einen handhabungssicheren Folienverband zur Verfügung zu stellen, der zudem Mittel umfasst, mit denen eine Anwendung desselben dokumentiert werden kann. Darüber hinaus besteht eine Aufgabe darin, einen Folienverband zur Verfügung zu stellen, der gezielt auf die vorgesehene Fläche aufgebracht werden kann, ein therapeutisches Mittel wie beispielsweise eine Kanüle fixieren kann und die Dokumentation des Gebrauchs des Folienverbandes oder der therapeutischen Maßnahme selbst im Routinebetrieb in einfacher Form ermöglicht und sicherstellt. Dabei soll der Folienverband ohne jede Form- oder Größeneinschränkung universell einsetzbar sein.

Gelöst wird diese Aufgabe durch einen Folienverband umfassend einen Polymerfilm und zur leichteren Handhabung des Folienverbandes ein Applikationssystem, wobei das Applikationssystem auf einer ersten Seite des Polymerfilms angeordnet ist und mindestens eine Stützfolie umfasst, an der zusätzlich mindestens eine Griffleiste angeformt ist, und wobei die Stützfolie eine erste, dem Polymerfilm zugewandte Seite und eine zweite, dem Polymerfilm abgewandte Seite aufweist und die zweite Seite der Stützfolie mindestens einen adhäsiven Bereich aufweist, und wobei die Stützfolie mittels des adhäsiven Bereiches direkt in eine Patienten- oder Dokumentationsmappe klebbar ist.

In einer zweiten bevorzugten Ausführungsform weist die Stützfolie einen adhäsiven Bereich auf, der mit einem Abdeckmittel, insbesondere mit einem streifenförmigen Abdeckmittel, abgedeckt ist.

Ein Vorteil eines Folienverbands mit einem solchen Applikationssystem besteht darin, dass die Stützfolie gleichzeitig auch als Dokumentationsmittel verwendet werden kann. Diese Dokumentation ist hilfreich im Umgang mit Folienverbänden, da die Folienverbände meist über einen Zeitraum von 7 bis 10 Tagen Anwendung finden. Im Gebrauch eines erfindungsgemäßen Folienverbandes kann die während der Applikation entfernte Stützfolie, durch und mittels des adhäsiven Bereiches direkt oder nach Entfernen des Abdeckstreifens in eine Patienten- oder Dokumentationsmappe geklebt werden. Somit wird in besonders einfacher Art ein Folienverband bereitgestellt, der durch die Griffleiste besonders leicht zu applizieren ist, und der dem Anwender gleichzeitig ein Mittel zur Dokumentation der Anwendung des Folienverbandes an die Hand gibt. Darüber hinaus ist eine eindeutige Dokumentation möglich, um ein zu frühes oder zu spätes Wechseln des Folienverbandes zu verhindern.

Als Applikationssystem soll bei einer erfindungsgemäßen Ausführung alles verstanden sein, das der leichteren Applikation des Polymerfilms dienen kann und mindestens eine Stützfolie und zusätzlich zu dieser Stützfolie zumindest eine Griffleiste umfasst, die an dieser Stützfolie angeformt ist. Unter Anformung soll hier das Verbinden zweier gleicher oder zweier verschiedener Materialien verstanden sein, die mit Hilfe von Klebemitteln, Druck, thermischer Energie, Ultraschallverfahren oder sonstiger Verfahren miteinander lösbar oder unlösbar verbunden sind. Die Griffleiste ist also vorliegend immer eine zusätzliche Materialkomponente, wobei die Griffleiste immer gleichzeitig mit mindestens einer Stützfolie von einem Polymerfilm entfernbar ist. Des Weiteren soll des leichteren Verständnisses wegen im Zusammenhang mit der vorliegenden Erfindung unter einem Film oder Polymerfilm immer der eigentlich zu applizierende Film oder Polymerfilm wie zum Beispiel ein Wundverband verstanden sein; dagegen soll unter einer Folie oder Polymer- bzw. Stützfolie immer ein Teil des Applikationssystems verstanden sein, das heißt die Unterscheidung zwischen Film und Folie ist hier lediglich auf die Funktion der Bestandteile zu verstehen. Hinsichtlich des Materials soll, wie im Sprachgebrauch üblich, kein Unterschied zwischen einem Film und einer Folie verstanden sein. Unter Dokumentation soll das Zusammentragen und zeitweise archivieren von Informationen verstanden sein.

Hinsichtlich der Ausgestaltung des adhäsiven Bereichs auf der Stützfolie sind dem Fachmann keine engen Grenzen gesteckt. Dieser adhäsive Bereich kann beispielsweise durch einen einfachen Streifen- oder Punktauftrag eines drucksensitiven Haftklebstoffes erfolgen. Besonders vorteilhaft sind solche Klebstoffe, die ein Wiederablösen beziehungsweise ein Neupositionieren der Stützfolie in einer Patientenmappe ermöglichen. Es kann aber auch ein doppelseitiges Haftklebeband Verwendung finden, wobei das Haftklebeband mittels der ersten haftklebenden Fläche nichtlösbar mit der Stützfolie verbunden ist und die der ersten Seite gegenüberliegende, haftklebende zweite Seite den adhäsiven Bereich der Stützfolie bildet. Bei dieser Ausgestaltung hat sich als besonders praktikabel herausgestellt, wenn die zweite Seite des doppelseitigen Klebebandes besonders gut auf Papier haftet. Ganz besonders bevorzugt sind solche Klebstoffe zu verwenden, die eine Klebkraft von 0,05 bis 0,5 N/ 5mm (gemessen nach DIN EN 1939) aufweisen.

In einer bevorzugten Ausgestaltung der Erfindung kann vorgesehen sein, dass die Griffleiste mittels des adhäsiven Bereichs auf der Stützfolie aufgebracht ist. Die Griffleiste bildet in dieser Ausgestaltung den Abdeckstreifen. Hierbei ist einerseits sicherzustellen, dass die Griffleiste, ohne die Stützfolie zu deformieren oder zu zerstören, von der Stützfolie entfernt werden kann, und andererseits die Haftklebrigkeit zwischen der Griffleiste und der Stützfolie ausreicht, um die Stützfolie mittels der Griffleiste von der Polymerfolie vollständig abzulösen. Darüber hinaus muss beispielsweise ein Adhäsiv, das dem adhäsiven Bereich aufgebracht ist und diesen somit bildet, nach dem Entfernen der Griffleiste vollständig auf der Stützfolie verbleiben, damit die Stützfolie ohne weitere Modifikation direkt in eine Patentenmappe einklebbar ist. Falls der Abdeckschicht ein von der Griffleiste verschiedenes Material ist, müssen diese Eigenschaften ebenfalls erfüllt sein. Als Abdeckstreifen kann hierfür jede/ jedes handelsübliche Silikonfolie oder -papier sowie eine/ ein mit einer Fluorverbindung beaufschlagte/ beaufschlagtes Folie oder Papier verwendet werden.

Zur universellen Anwendung der Stützfolie ist vorgesehen, dass die Stützfolie selbst auf der dem Polymerfilm zugewandten, ersten Seite mit einem üblichen Schreibgerät wie zum Beispiel einem Permanet-Marker beschreibbar ist. So kann zum Beispiel eine Kennzeichnung des Anwenders zur behandelten Person oder zum Zeitpunkt der Anwendung auf der Stützfolie dokumentiert werden. Insbesondere soll die erste Seite beschreibbar sein, da diese Seite nach dem Einkleben in beispielsweise eine Patientenmappe oben liegt und somit auch ein Beschreiben nach dem Einkleben in eine Patientenmappe möglich ist.

Der Übersicht wegen kann die Stützfolie mit einem Informationen vermittelnden Aufdruck versehen sein, der eine Dokumentation der Anwendung erleichtert. Dieser Aufdruck kann beispielsweise ein Abbildung der Worte Datum, Anwender, Patient oder die Namensnennung des Herstellers oder die Produktbezeichnung sein. Der Aufdruck kann auf der dem Polymerfilm zugewandten ersten Seite der Stützfolie oder auf der zweiten Seite der Stützfolie aufgebracht sein. Falls der Aufdruck auf der zweiten Seite erfolgt, erfolgt der Aufdruck insbesondere spiegelverkehrt, damit dieser Aufdruck nach dem Einkleben in eine Patientenmappe lesbar ist.

Es kann weiterhin vorgesehen sein, dass als Stützfolie eine mehrlagige Folie oder ein Folienverbund verwendet wird. Diese mehrlagigen Folien besitzen den Vorteil, dass ein eventueller Aufdruck auf einer ersten Lage der Folie durch eine zweite Lage Folie abgedeckt werden kann. Somit kann eine Markierung mittels eines Permanent-Markers auf einer äußeren Oberfläche der Folie erfolgen, die eine gleichmäßige und einheitliche Form aufweist. Es kann weiterhin vorgesehen sein, dass die Folie als ganzes eingefärbt ist, insbesondere derart, dass sie gleichwohl transparent bzw. transluzent ist. Besonders bevorzugte sind transparente oder transluzente Stützfolien.

Damit ist auch die Verwendung einer Stützfolie eines Folienverbandes zur Dokumentation der Anwendung des Folienverbandes Gegenstand der vorliegenden Erfindung.

In einer weiteren besonders bevorzugten Ausführungsform ist vorgesehen, dass der Folienverband einen Polymerfilm aufweist, der mindestens einen ersten stützfolienfreien Bereich aufweist. Durch den ersten stützfolienfreien Bereich ist die durch die Stützfolie abgedeckte Fläche des Polymerfilms somit kleiner ist als die Fläche der ersten Seite des Polymerfilms. Insbesondere weist der Folienverband eine oder mehrere Stützfolien auf, deren Auflagefläche insgesamt weniger als 97 % und insbesondere weniger als 94 % der Fläche der ersten Seite des zu applizierenden Polymerfilms entspricht.

Ein Vorteil eines solchen Folienverbandes mit Applikationssystem besteht darin, dass der stützfolienfreie Bereich des Polymerfilms, das heißt der nicht durch eine Stützfolie abgedeckte Bereich, aufgrund der leichteren Biegsamkeit im Vergleich zum Polymerfilm mit Stützfolie, während der Applikation des Folienverbandes als Gelenk fungieren kann. Somit kann als Stützfolie auch ein relativ steifes Stützmaterial zum Einsatz gebracht werden, wobei gleichzeitig eine passgenaue Applikation gewährleistet ist. Beim Stand der Technik mit einer einteiligen Stützfolie dagegen, die gleich groß wie der Polymerfilm ist oder bei einer mehrteiligen Stützfolie, die insgesamt gleich groß wie der Polymerfilm ist, muss die Wahl des Stützmaterials auf relativ flexible Materialien eingeschränkt werden, um eine passgenaue Applikation des Polymerfilms zu gewährleisten. Ein weiterer Vorteil besteht in der Materialeinsparung der für die Stützfolien verwendeten Materialien.

Insbesondere kann ein erster stützfolienfreier Bereich an einem Rand des Polymerfilms angeordnet sein. Als Rand soll hier jeder Bereich einer Fläche verstanden sein, der sich von der Kante einer Fläche in das Innere einer Fläche erstreckt, wobei die flächenhafte Ausdehnung des Randes kleiner ist als 50% der gesamten Fläche. Hierdurch wird ein Folienverband bereitgestellt, der vorteilhafter Weise einen Bereich aufweist, der eine durch den Polymerfilm selbst vorgegebene Flexibilität aufweist und eine leichte Erstfixierung des zu applizierenden Films gewährleistet, wobei gleichzeitig durch die Stützfolien auf den weiteren Bereichen eine sichere Handhabung gewährleistet ist. Hierbei hat sich als besonders gut handhabbar und anwendungssicher herausgestellt, wenn eine der Stützfolien in mindestens einem Punkt ihrer äußeren Kante einen Abstand von der äußeren Kante des Polymerfilms von mindestens 2 mm, insbesondere mindestens 3 mm und ganz besonders mindestens 5 mm besitzt. Besonders bevorzugt ist ein Abstand, der in jedem Punkt der Kante der Stützfolie einen gleichen Abstand von mindestens 2 mm, insbesondere mindestens 3 mm und ganz besonders mindestens 5 mm zu der äußeren Kante des Polymerfilms aufweist.

In einer weiteren Ausgestaltung der Erfindung ist vorgesehen, dass eine Griffleiste einen nicht durch die Stützfolie abgedeckten Bereich des Polymerfilmes vollständig überfängt. In diesem Fall kann das Applikationssystem insgesamt dieselbe Größe wie der Polymerfilm aufweisen. Hierbei ist mit derselben Größe, eine Größe hinsichtlich der Auflagefläche gemeint, das heißt die Umfangsbegrenzungen des Applikationssystems und des Polymerfilmes sind bündig. Durch dieselbe Größe des Applikationssystems und des Polymerfilms und dadurch, dass die Griffleiste lediglich an der Stützfolie angeformt ist und keine Verbindung zu dem Polymerfilm aufweist, ist gewährleistet, dass neben der bereits geschilderten hohen Flexibilität in dem nicht durch die Stützfolie abgedeckten Bereich zudem der gesamte Film abgedeckt ist und somit auch vor und während der Applikation vollständig geschützt ist.

In einer bevorzugten Ausführungsform eines erfindungsgemäßen Folienverbandes ist vorgesehen, dass eine Griffleiste eine zum Ergreifen durch den Benutzer bestimmte Grifffläche, vorzugsweise ausgeführt als Hintergriffsmittel, von mindestens 2 cm², insbesondere mindestens 5 cm² und ganz besonders bevorzugt von mindestens 7 cm² aufweist.

In einer weiteren Ausführung dieser Erfindung umfasst das Applikationssystem mindestens zwei Stützfolien, die in einer Ebene parallel zu dem Polymerfilm aufgebracht werden. In einer solchen Ausgestaltung der Erfindung kann ein erster stützfolienfreier Bereich zwischen der ersten und der zweiten Stützfolie liegen. Der Abstand dieser beiden Stützfolien zueinander beträgt dabei vorzugsweise in jedem Punkt mindestens 2 mm, insbesondere 3 mm und ganz besonders mindestens 5 mm. Besonders bevorzugt ist ein Applikationssystem, das zwei Stützfolien aufweist, die in jedem Punkt den gleichen Abstand zueinander aufweisen.

Insbesondere ist bei der Ausgestaltung des Folienverbandes mit zwei Stützfolien vorgesehen, dass jede Stützfolie jeweils eine Griffleiste umfasst. Dabei werden eine erste Griffleiste der ersten Stützfolie und eine zweite Griffleiste der zweiten Stützfolie zugeordnet.

In einer bevorzugten Ausführungsform dieses erfindungsgemäßen Folienverbandes mit zwei Stützfolien ist vorgesehen, dass die erste Griffleiste eine zum Ergreifen durch den Benutzer bestimmte Grifffläche, vorzugsweise ausgeführt als Hintergriffsmittel, aufweist und die erste Griffleiste mit dieser Grifffläche die zweite Griffleiste zumindest abschnittsweise, insbesondere aber vollständig überfängt.

Durch diese Anordnung der Griffleisten ist in besonders einfacher Weise gewährleistet, dass der Anwender in jedem Fall nur die zu oberst liegende erste Griffleiste als erste Griffleiste betätigen kann und somit eine erste Stützfolie als erste Folie von dem Polymerfilm entfernen kann. Erst im zweiten Schritt kann der Anwender daraufhin eine zweite Stützfolie mit Hilfe der zweiten Griffleiste entfernen. Es ist somit eine Reihenfolge bei der Entfernung der Stützfolien vorgegeben und ein besonders handhabungssicherer Folienverband bereitgestellt.

Vorzugsweise beträgt die Größe der Grifffläche der ersten Griffleiste mindestens 2 cm², insbesondere mindestens 5 cm² und ganz besonders bevorzugt von mindestens 7 cm². Als besonders handhabungssicher hat sich herausgestellt, wenn die erste Griffleiste eine freie Grifffläche von mindestens 2 cm², insbesondere 4 cm² und ganz besonders bevorzugt von 6 cm² aufweist. Diese freie Grifffläche ist vorliegend der Teil der Grifffläche, der über die zweite Griffleiste seitlich übersteht.

Als besonders handhabungssicher hat sich dabei herausgestellt, wenn die erste Griffleiste sowohl den stützfolienfreien Bereich des Polymerfilms als auch die zweite Griffleiste vollständig überfängt.

Falls der Folienverband ein Applikationssystem mit zwei Stützfolien aufweist und ein erster stützfolienfreier Bereich zwischen den Stützfolien vorgesehen ist, kann getrennt von diesem ersten stützfolienfreien Bereich ein zweiter stützfolienfreier Bereich vorgesehen sein. Dieser zweite Bereich kann weiterhin vorzugsweise durch eine Griffleiste überfangen sein. Es kann aber auch vorgesehen sein, dass dieser zweite Bereich weder von einer Stützfolie noch von einer Griffleiste überdeckt wird. Vorzugsweise ist dieser zweite stützfolienfreie Bereich des Polymerfilms an einem Rand des Folienverbandes angeordnet. Auf diese Weise weist der Folienverband sowohl ein Gelenk innerhalb des Verbandes auf als auch einen Bereich zur Erstfixierung auf.

Falls ein Applikationssystem vorgesehen ist, das mehr als zwei Stützfolien umfasst, kann jeder Stützfolie eine Griffleiste zugeordnet sein. In einer besonders bevorzugten Ausführung kann auch eine Griffleiste zwei Stützfolien zugeordnet sein. Insbesondere kann in einem Folienverband mit drei Stützfolien eine Griffleiste zwei Stützfolien zugeordnet sein. Durch diese Anordnung bzw. Zuordnung der Griffleisten auf den Stützfolien können zwei separate Stützfolien in einem Arbeitsschritt entfernt werden.

Ein erfindungsgemäßer Folienverband kann in jeder geometrischen Form ausgestaltet sein wie beispielsweise oval, quadratisch, rechteckig, fünfeckig, sechseckig oder anderen denkbaren Formen. Insbesondere kann ein erfindungsgemäßer Folienverband auch in Rechteckiger Form gestaltet sein. Gemäß einer weiteren bevorzugten Ausführungsform kann ein erfindungsgemäßer Folienverband auch von einem Randbereich zu einem mittleren Bereich einen Einschnitt aufweisen. In einer solchen Ausgestaltung ist ein vorliegender Folienverband besonders gut als so genanntes Kanülen- oder Katheterpflaster zu verwenden.

Als Stützfolien sind besonders transparente oder transluzente Folienmaterialien vorgesehen. Alternativ können jedoch auch opake oder undurchsichtige Folienmaterialien verwendet werden. Insbesondere kommen als Stützfolie solche Folien zur Verwendung, die aus Polyester, Polyethylen, Polypropylen, Polyvinylchlorid, Polystyrol, Polyamid, Polykarbonat, Celluloseester, Ethylenvinylacetat, Polyvinylacetat, Polyvinylalkohol und/oder Mischungen hiervon gefertigt werden. Besonders bevorzugt sind Stützfolien aus transparentem Polyester oder Polyethylen oder Polypropylen. Dabei hat es sich weiterhin als besonders vorteilhaft erwiesen, wenn die Dicken der Stützfolie so eingestellt werden, das diese eine Dicke von 15 bis 80 pm, insbesondere von 20 bis 60 µm und ganz bevorzugt von 20 bis 40 µm aufweisen.

Zur Herstellung einer Griffleiste können dieselben Materialien verwendet werden, die als Stützfolie zur Anwendung gebracht werden. In einer besonders bevorzugten Ausführungsform wird die Griffleiste aber aus einem Folienmaterial hergestellt, das flexibler ist als die Stützfolie. Falls ein Applikationssystem vorgesehen ist, das zwei oder mehr Stützfolien und zwei oder mehr Griffleisten umfasst, sind vorzugsweise alle Griffleisten aus einem Material gefertigt, das flexibler ist als jede Stützfolie. Hierdurch ist gewährleistet, dass die Griffleisten besonders leicht zu ergreifen sind. In einer weiteren besonders bevorzugten Ausführungsform mit zwei Griffleisten ist vorgesehen, dass die Griffleiste der ersten Stützfolie flexibler ist als die Griffleiste der zweiten Stützfolie. Dabei ist es weiterhin vorteilhaft, wenn die zweite Griffleiste vollständig von der ersten Griffleiste übergriffen wird.

Zusätzlich kann bei einem System mit zwei Griffleisten ein Aktivierungsmittel vorgesehen sein, dass zwischen der ersten und der zweiten Griffleiste angeordnet ist. Dieses Aktivierungsmittel kann zum Beispiel ein zusätzlicher adhäsiver Streifen sein, der zu beiden Seiten seiner Auflageflächen eine unterschiedliche Klebekraft aufweist. Bei der Anwendung eines solchen Folienverbandes kann dann zum Beispiel eine über der zweiten Griffleiste angeordnete erste Griffleiste ergriffen werden und mit dieser Griffleiste das Aktivierungsmittel und eine Stützfolie von dem Polymerfilm entfernt werden, wobei gleichzeitig die zweite Griffleiste so aktiviert wird bzw. aufgerichtet wird, dass diese von dem Anwender leichter greibar ist.

Als Polymerfilm können in einem erfindungsgemäßen Folienverband insbesondere Polymerfilme eingesetzt werden, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 µm, insbesondere 20 bis 40 µm und ganz besonders bevorzugt von 25 bis 30 µm aufweisen. Die Wasserdampfdurchlässigkeit des Polymerfilms in einem erfindungsgemäßen Folienverbande weist vorzugsweise mindestens 750 g/ m²/ 24 Std., insbesondere mindestens 1000 g/ m²/ 24 Std. und ganz besonders bevorzugt mindestens 2000 g/ m²/ 24 Std. auf (gemessen nach DIN 13726).

Auf der dem Applikationssystem gegenüberliegenden zweiten Seite des zu applizierenden Polymerfilms kann ein Klebemittel aufgebracht sein. Dieser Auftrag kann sowohl vollflächig als auch diskontinuierlich oder nur in bestimmten Bereichen erfolgen. Bei dem verwendeten Klebemittel kann es sich um einen üblichen Haftkleber, insbesondere um einen Acrylathaftkleber handeln. Ganz besonders bevorzugt handelt es sich um Hydrogelhaftkleber, insbesondere auf Basis von wässrigen Acrylaten.

Vorteilhafterweise beträgt das Flächengewicht des Klebemittels 20-100g/m², insbesondere 30-50 g/m², wobei das Klebemittel diskontinuierlich, vorzugsweise aber vollflächig aufgetragen sein kann.

Die Wasserdampfdurchlässigkeit des mit dem Klebemittel versehenen Polymerfilm beträgt vorzugsweise mindestens 1000 g/ m²/ 24 Std., besonders bevorzugt mindestens 1200 g/ m²/ 24 Std. und ganz besonders bevorzugt mindestens 2000 g/ m²/ 24 Std. (gemessen nach DIN EN 13726) aufweisen.

Gemäß einer Weiterführung der Erfindung kann der Folienverband auf der dem Applikationssystem gegenüberliegenden zweiten Seite des Polymerfilms vollflächig mit einem Haftklebemittel beschichtet sein und das Haftklebemittel mit einer Abdeckfolie oder einem Abdeckpapier geschützt sein. Als Abdeckschicht kann dabei jedes handelsübliche Silikonpapier oder -folie sowie eine mit einer Fluorverbindung beaufschlagtes Papier oder Folie verwendet werden.

Um einen anwendungssicheren Folienverband bereit zu stellen, muss eine genaue Abstimmung der verwendeten Materialien erfolgen. Insbesondere müssen die verwendeten Materialien hinsichtlich ihrer Release-Eigenschaften abgestimmt werden. Diese durch zusätzliche Mittel einstellbaren Release-Eigenschaften beruhen auf den Kräften, die zwischen zwei verwendeten Materialien herrschen. So kann zum Beispiel durch eine gezielte Oberflächenbehandlung eines Materials eine anziehende oder eine abstoßende Wirkung auf ein zweites mit diesem ersten Material zusammen zu bringendes Material eingestellt werden. Eine Oberflächenbehandlung, die eine anziehende Wirkung zwischen zwei Materialien hervorruft, kann zum Beispiel durch einen zusätzlichen adhäsiven Auftrag, eine statische Aufladung oder durch ein Verschmelzen der beiden zusammenzubringenden Materialien erfolgen. Eine abstoßende Wirkung kann zum Beispiel durch eine zusätzliche Schicht auf einem Material aus Silikon- oder Fluorverbindungen hervorgerufen werden. Als Release-Kraft (Abziehkraft) wird dabei eine solche Kraft verstanden, die notwendig ist zwei Materialien voneinander zu trennen (gemessen nach DIN 53530).

In einer weiteren Ausführungsform des erfindungsgemäßen Folienverbandes sind diese Release-Eigenschaften so eingestellt, dass die Abziehkraft, die notwendig ist um eine Abdeckfolie oder -papier von dem zu applizierenden Polymerfilm zu lösen, größer ist als die Abziehkraft, die notwendig ist, um die Stützfolie oder die Stützfolien von dem Polymerfilm zu trennen.

Bei einer Ausgestaltung des Folienverbandes mit zwei Stützfolien sind die Release-Eigenschaften bevorzugt so eingestellt, dass die Abziehkraft, die notwendig ist um die erste Stützfolie von dem zu applizierenden Polymerfilm zu lösen, gleich groß ist wie die Abziehkraft, die zum Lösen der zweiten Stützfolie notwendig ist.

Weiterhin bevorzugt sind bei einem Folienverband mit zwei Stützfolien und zwei Griffleisten die Release-Eigenschaften so eingestellt, das die Abziehkraft, die notwendig ist, um die erste Griffleiste von der zweiten Griffleiste oder die zweite von der ersten Griffleiste zu lösen, geringer ist als die Ablösekraft, die notwendig ist, um die Stützfolie von dem zu applizierenden Polymerfilm zu lösen.

Bei einer weiteren Ausgestaltung des Folienverbandes mit zwei Stützfolien sind die Release-Eigenschaften bevorzugt so eingestellt, das die Abziehkraft, die notwendig ist, um die erste Stützfolie von dem zu applizierenden Polymerfilm zu lösen, größer ist als die Abziehkraft, die zum Lösen der ersten Griffleiste von der zweiten Griffleiste notwendig ist.

Die Haftung der Stützfolie auf dem Polymerfilm ist vorzugsweise nur 0,01 bis 0,5 N/ 25 mm, ganz besonders bevorzugt 0,01 bis 0,1 N/ 25 mm, gemessen nach DIN 53530. Hierzu, wird das Stützmaterial vorzugsweise direkt bei der Herstellung des Polymerfilms auf den Polymerfilm aufgebracht, bzw. der Polymerfilm direkt auf dem Stützmaterial erzeugt. Hierbei können alle üblichen Verfahren zur Filmherstellung angewendet werden zum Beispiel durch Giessen, Rakeln, Extrudieren oder andere bekannte Methoden der Film- oder Folienherstellung. Falls notwendig, kann das Stützmaterial auf der Beschichtungsseite aufgeraut oder einer anderen haftungsfördernden Behandlung unterworfen werden. Auch eine adhäsionsfördernde Beschichtung kann vorteilhaft sein. Wichtig dabei ist, dass die Haftung des Polymerfilms auf der zu applizierenden Oberfläche wesentlich größer ist als die Haftung einer Stützfolie an dem Polymerfilm.

In einer besonderen Ausgestaltung der Erfindung ist vorgesehen, dass ein Folienverband umfassend einen Polymerfilm mit einem Applikationssystem in einer Verpackung angeordnet ist. Insbesondere ist dabei vorgesehen, dass die Verpackung eine sterile Verpackung ist. Es kann auch vorgesehen sein, das separat neben dem Folienverband ein Wundpad in der Verpackung eingebracht ist.

Nachstehend wird die Erfindung unter Bezugnahme auf die Zeichnung erläutert. In den Zeichnungen zeigt:
- Figur 1:: eine erste Ausgestaltung eines Folienverbandes, in Aufsicht
- Figur 2:: die in Figur 1 wiedergegebene Ausführungsform eines Folienverbandes, im Querschnitt
- Figur 3:: eine Ausgestaltung der Stützfolie als Informationsträger
- Figur 4:: eine weitere Ausführungsform eines Folienverbandes, in Aufsicht
- Figur 5:: die in Figur 4 wiedergegebene Ausführungsform eines Folienverbandes, im Querschnitt
- Figur 6:: eine weitere Ausführungsform eines Folienverbandes, in Aufsicht
- Figur 7:: die in Figur 6 wiedergegebene Ausführungsform eines Folienverbandes, im Querschnitt
- Figur 8a - c:: eine Ausführungsform bei der Verwendung, im Querschnitt

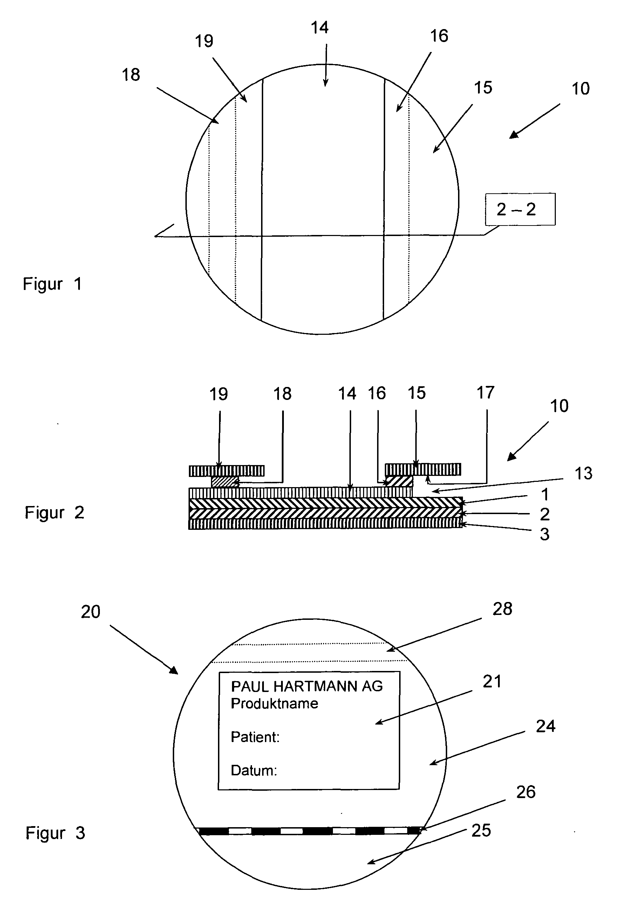

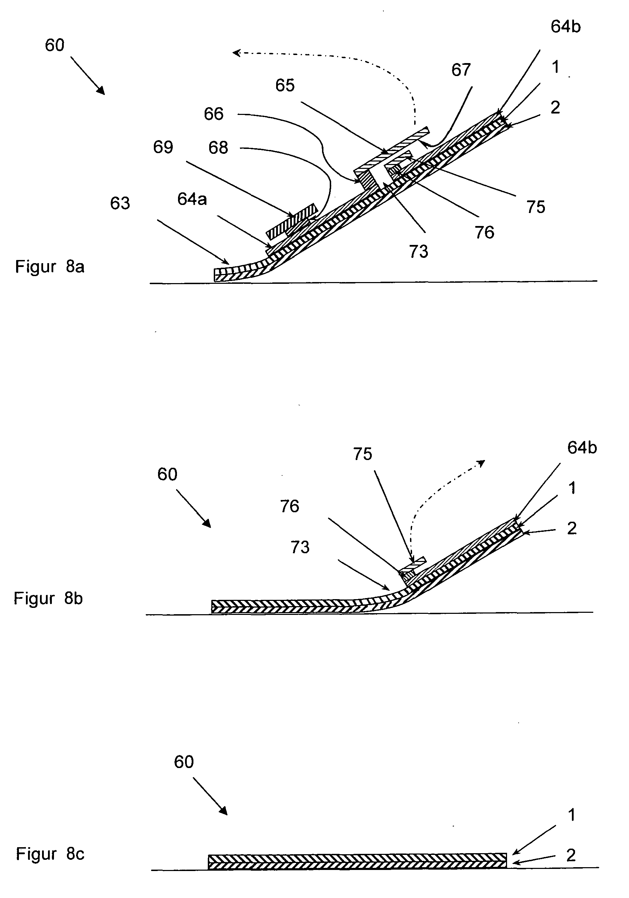

Mit Figur 1 und Figur 2 ist eine erste Ausführungsform eines erfindungsgemäßen Folienverbandes gezeigt. Hier und im Folgenden sind alle gepunkteten Markierungen als Grenzen von Bauteilen zu verstehen, die unterhalb der mit durchzogenen Linien dargestellten Bauteile liegen. Der Folienverband (10) liegt als Ganzes in kreisrunder Form vor. Er besteht aus einem transparenten Polymerfilm (1) der auf seiner ersten Seite mit einem Applikationssystem abgedeckt ist. Auf der dem Applikationssystem abgewandten zweiten Seite ist ein adhäsives Klebemittel (2) aufgebracht, das durch eine Abdeckschicht (3) geschützt ist. Das Applikationssystem besteht aus einer ebenfalls transparenten Stützfolie (14), die den Polymerfilm (1) bis auf den stützfolienfreien Randbereich (13) vollständig überdeckt. Weiterhin weist die Stützfolie (14) einer Griffleiste (15) auf, die mittels eines Adhäsivs (16) auf der Stützfolie fest verbunden aufgebracht ist. Die Griffleiste ist in keinem Punkt mit dem Polymerfilm verbunden und überdeckt den stützfolienfreien Randbereich (13) vollständig. Durch die Grifffläche (17) kann der Anwender die Griffleiste (15) besonders leicht ergreifen. Damit ist das Applikationssystem als Ganzes gleich groß wie der Polymerfilm (1) und weist dieselbe Umfangsbegrenzung auf, wie der Polymerfilm. Darüber hinaus weist die Stützfolie auf der dem Polymerfim abgewandten Seite einen adhäsiven Bereich (18) auf, der mittels eines Abdeckstreifens (19) aus einem silikonisierten Papier abgedeckt ist. Bei der Verwendung kann der Anwender nach erfolgter Applikation des Folienverbandes (vgl. Figuren 8a - 8c) den silikonisierten Abdeckstreifen (19) entfernen und die Stützfolie zur Dokumentation der Anwendung des Folienverbandes in eine Patientenmappe einkleben.

Mit Figur 3 ist ein Beispiel einer Stützfolie (20) in Aufsicht gezeigt, wobei mit dieser Abbildung die Aufsicht der in dem Folienverband dem Polymerfilm zugewandten ersten Seite wiedergegeben ist. Diese Stützfolie besteht aus einer einlagigen, transluzenten Polyethylen-Polymerfolie (24), an die mittels Ultraschallnaht (26) eine Griffleiste (25) aus einem farbigen, transluzenten Polyethylenmaterial aufgebracht ist. Auf der ersten Seite der Stützfolie ist ein Aufdruck aufgebracht. Dieser Aufdruck kennzeichnet ein Informationsfeld (21), in das Daten zur Anwendung des Produkts übernommen werden können. Die Polymerfolie kann mittels eines adhäsiven Bereiches (28) auf der Unterseite der Folie in eine Patientenmappe eingeklebt werden.

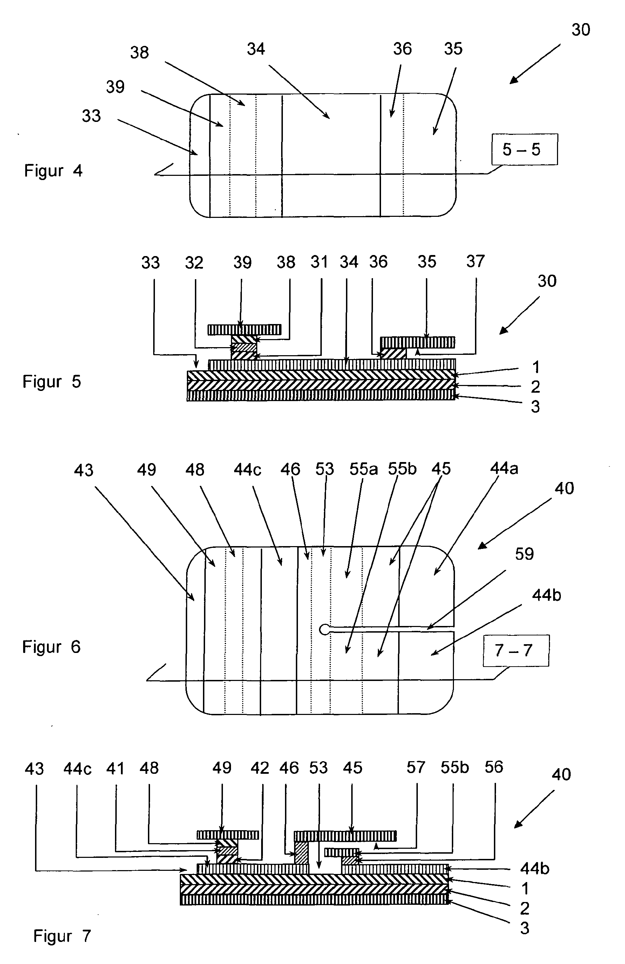

Mit Figur 4 und Figur 5 ist eine zweite Ausführungsform der vorliegenden Erfindung gezeigt. Dieser Folienverband (30) ist von rechteckiger Grundform und besteht wie die erste Ausführung aus einem Polymerfilm (1), einem auf den Polymerfilm aufgebrachten vollflächigen Klebstoffauftrag (2) und einer diesen Klebstoff abdeckenden Abdeckschicht (3). Auf der ersten Seite des Polymerfilms weist dieser ein Applikationssystem auf, das eine Stützfolie (34), eine mittels eines Adhäsivs (36) auf der Stützfolie angeordnete Griffleiste (35) und einen adhäsiven Bereich (38) umfasst. Der adhäsive Bereich (38) ist durch einen zweiseitig klebenden Streifen realisiert, der aus einem ersten Adhäsiv (31), einem Trägermaterial (32) und einem zweiten Adhäsiv (38) besteht. Durch das erste Adhäsiv wird das Trägermaterial (41) fest mit der Stützfolie verbunden. Das mit einem silikonisierten Papier (39) abgedeckte Adhäsiv (38) bildet den adhäsiven Bereich der Stützfolie (34) mittels dessen die Stützfolie in eine Patientenmappe oder ähnlichem fixierbar ist und somit eine Dokumentation der Anwendung des Folienverbandes erfolgen kann. Die Stützfolie ist insgesamt kleiner als der Polymerfilm, das heißt, dass die Auflagefläche der Stützfolie 95% der Auflagefläche des Polymerfilms entspricht. Dadurch, dass der stützfolienfreie Bereich einen Randbereich des Polymerfilms bildet, kann dieser Bereich bei der Applikation des Folienverbandes zur Erstfixierung verwendet werden.

Mit Figur 6 und Figur 7 ist ein weiterer Folienverband (40) wiedergegeben. Dieser Folienverband kann als Kanülen- oder Katheterpflaster verwendet werden. Der Folienverband weist eine rechteckige Grundform auf, deren kurze Seite parallel zur langen Seite eine Aussparung (59) aufweist. Durch diese Aussparung erhält der Folienverband zwei voneinander unabhängige Bereiche, die über einen dritten Bereich miteinander verbunden sind und die bei der Fixierung, zum Beispiel einer Kanüle jeweils auf einer Seite der Kanüle auf einer Oberfläche fixiert werden können. Der Folienverband weist einen Polymerfilm (1), eine Acrylat-Klebeschicht (2) und eine die klebende Schicht abdeckende Abdeckschicht (3) auf. Auf der der klebenden Schicht abgewandten ersten Seite des Polymerfilms ist ein Applikationssystem angeordnet. Dieses Applikationssystem umfasst drei Griffleisten (45, 55a, 55b), die mittels dreier Adhäsive (46, 56a, 56b - 56a ist nicht dargestellt) auf drei Stützfolien (44a, 44b, 44c) aufgebracht sind. Die erste Griffleiste (45), die der ersten Stützfolie (44c) zugeordnet ist, überfängt sowohl den zentralen Bereich (53) des Polymerfilmes, der nicht durch Stützfolien abgedeckt ist, als auch die zweite und die dritte Griffleiste (55a, 55b). Der äußere Teil der Grifffläche der Griffleiste (45) ragt seitlich als freie Grifffläche (57) über die zweite und dritte Griffleiste (55a, 55b) hinaus, so dass eine Reihenfolge beim Entfernen derselben vorgegeben ist. Darüber hinaus ist die Griffleiste (45) über einen Teil ihrer Fläche durch die Ausspaarung (59) eingeschnitten. Die zweite und die dritte Griffleiste (55a, 55b) überfangen ebenfalls einen zentralen Bereich des Polymerfilms (53), der nicht durch eine Stützfolie abgedeckt ist. Auf der ersten Stützfolie (45) ist ein zweiseitig haftendes Klebeband aufgebracht, dass aus einem ersten Adhäsiv (42), einem Trägermaterial (41) und einem zweiten Adhäsiv (48) besteht. Das mit einem silikonisierten Papier (49) abgedeckte Adhäsiv (48) bildet den adhäsiven Bereich der ersten Stützfolie (44c), mittels dessen die Stützfolie (44c) in eine Patientenmappe oder ähnlichem fixierbar ist. Somit kann eine Dokumentation der Anwendung des Folienverbandes erfolgen.

Die Stützfolien (44a, 44b, 44c) bedecken insgesamt eine Fläche des Polymerfilms, die 92% der Fläche der ersten Seite des Polymerfilms beträgt, da neben dem zentralen Bereichen (53), der nicht durch Stützfolien abgedeckt ist, auch in dem Randbereich (43) keine Stützfolie angeordnet ist. Dieser Randbereich kann nach dem Entfernen der Abdeckschicht zur Erstfixierung verwendet werden. Es kann weiterhin auch ein Aktivierungsmittel zwischen den beiden Griffleisten vorgesehen sein. Dieses Aktivierungsmittel kann ein doppelseitiges Klebeband, das zur ersten Griffleiste (45) eine höhere Klebkraft aufweist als zur zweiten Griffleiste (55a, 55b). Damit wird bei der Entfernung der Stützfolie (44c) mittels der ersten Griffleiste (45) die darunter liegende zweite und dritte Griffleiste (55a, 55b) aufgerichtet, bevor die Klebkraft zwischen der zweiten und der dritten Griffleiste (55a, 55b) und dem Aktivierungsmittel aufgehoben wird. Die zweite und die dritte Griffleiste ist somit im nächsten Schritt zur Entfernung der zweiten und der dritten Stützfolie (44a, 44b) leichter greifbar.

Bei der Anwendung eines erfindungsgemäßen Folienverbandes (10, 30, 40, 50) ist nun vorgesehen, zuerst die Abdeckschicht (3) von der Klebstoffschicht (2) zu entfernen. Wie in den Figuren 8a bis 8c gezeigt ist, kann bei einem Folienverband mit zwei stützfolienfreien Bereichen daraufhin, um einen Folienverband (40, 60) zu applizieren, ein erster stützfolienfreier Randbereich (43, 63) fixiert werden. Durch die hohe Flexibilität des Polymerfilms im stützfolienfreien Bereich ist dies sehr gut möglich. Im einem weiteren Schritt kann der Anwender dann durch die vorgegebene Anordnung der Griffleisten (45, 55, 65, 75), die mittels adhäsiver Klebstoffe (46, 56, 66, 76) auf den jeweiligen Stützfolien fest aufgebracht sind, den zu applizierenden Polymerfilm (1) zielgenau platzieren. Durch den zweiten stützfolienfreien Bereich (53, 73) weist der Folienverband eine Art Gelenk auf, das eine faltenfreie Applikation gestattet. Die Stützfolien (44a, 44b, 44c, 64a, 64b) können nacheinander während der Applikation oder nacheinander nach erfolgter Applikation des Polymerfilms entfernt werden, wobei zunächst die erste Griffleiste (45, 65) über ihre freie Grifffläche (57, 67) ergriffen und damit zuerst die erste Stützfolie (44a, 64a) abgezogen werden kann.

### Ausführungsbeispiel 1:

Der Folienverband weist eine rechteckige Grundform mit einer Kantenlänge von 105 x 85 mm auf (Auflagefläche 89,25 cm²). Er umfasst einen transparenten Polyurethanfilm, der auf seiner in Gebrauch körperzugewandten Seite mit einem druckempfindlichen Acrylat-Klebstoff beschichtet ist. Der Klebstoff ist in einem vollflächigen Auftrag in einer Menge von ca. 25 g/ m² auf den ca. 25 µm dicken Polymerfilm (gemessen bei einem Prüfdruck von 0,5 kPa) aufgebracht. Der Polymerfilm weist zusammen mit dem Klebstoff eine Wasserdampfdurchlässigkeit von ca. 1200 g/ m²/ 24 Std. auf (gemessen nach DIN EN 13726). Ein solcher Polymerfilm ist unter dem Handelsnamen Inspire 1305 bei der Fa. InteliCoat Technologies, Wrexham Industrial Estate, Wrexham LL13 9UF, UK, erhältlich. Die klebende Seite dieses Polymerfilms ist mit einem silikonisierten Abdeckpapier erhältlich unter dem Handelsnamen Separacon 980-60 bei der Fa. Maria Soell GmbH & Co. KG, Frankenstrasse 45, D-63667 Nidda-Eichelsdorf, abgedeckt. Der Folienverband ist als Kanülen- oder Katheterpflaster gefertigt und zeigt den mit Figuren 6 und 7 prinzipiell wiedergegebnen Aufbau. Auf der anderen, in Gebrauch körperabgewandten Seite des Polymerfilmes ist ein Applikationssystem angeordnet, das aus drei Stützfolien aus Polyethylenterephthalat (PET) besteht, auf denen jeweils eine Griffleiste angeordnet ist. Der vorliegende Folienverband enthält einen Polymerfilm, der zwei voneinander getrennte Bereiche aufweist, die durch keine Stützfolie und keine Griffleiste abgedeckt ist. Zum einen ist dies ein Randbereich (vgl. Fig. 6 oder 7, (43)) und ein zentraler Bereich (vgl. Fig. 6 und 7, (53)) des Polymerfilms. Der Randbereich ist auf der kurzen Seite des Rechtecks angeordnet und weist eine gleichförmige Breite von 6 mm auf. Die beiden durch den Schlitz getrennten Stützfolien sind gleich groß und weisen eine Kantenlänge von 42 x 42 mm auf (Auflagefläche: 2 x 17,6 cm² = 35,3 cm²). Der Abstand der beiden Folien beträgt in jedem Punkt ihrer zueinander parallel liegenden gleich langen Kanten ca. 1 mm. Dieser Abstand entspricht dem mit Figur 6 wiedergegebenen Spalt (59). Die dritte Stützfolie weist eine Auflagefläche von 85 x 50 mm auf (Auflagefläche: 42,5 cm²) und ist durch den zentralen Bereich (53) an der langen Seite 10 mm von den beiden anderen Stützfolien beabstandet. Damit ergibt sich insgesamt für die drei Stützfolien eine Auflagefläche von ca. 87 % bezogen auf Fläche der ersten Seite des Polymerfilms. Die Stützfolien sind aus einem 30 µm dicken PET-Folie gefertigt (gemessen bei einem Prüfdruck von 0,5 kPa). Auf jeder Stützfolie ist mittels eines Acrylat-Klebstoffes eine Griffleiste aufgeklebt. Die Griffleisten weisen im Querschnitt betrachtet eine Anordnung auf, wie sie mit Figur 7 wiedergegeben ist, wobei die erste mit Bezugszeichen (45) skizzierte Griffleiste eine Größe von 85 x 30 mm aufweist und über die gesamte Breite (85 mm) mit der zugehörigen Stützfolie verbunden ist. Die zweite mit Bezugszeichen (55a) und die dritte mit Bezugszeichen (55b) wiedergegeben Griffleisten weisen jeweils eine Größe von 42 x 15 mm auf. Alle Griffleisten sind jeweils über einen 4 mm breiten adhäsiven Verbindungsstreifen mit der jeweiligen Stützfolie verbunden und sind aus einer 20 µm dicken transparenten Polyesterfolie gefertigt. Somit hat die erste Griffleiste eine Grifffläche, deren gleichförmige Breite 26 mm beträgt. Die Größe der Grifffläche der ersten Griffleiste beträgt 85 x 26 mm = 22,1 cm². Die gleichförmige Breite der Grifffläche der zweiten und der dritten Griffleiste beträgt jeweils 11 mm. Somit beträgt die Größe der Grifffläche der zweiten oder der dritten Griffleiste 42 x 11 mm = 4,6 cm². Die gleichförmige Breite des über die zweite oder dritte Griffleiste überstehenden Teils der ersten Grifffläche, also die Breite der freien Grifffläche der ersten Griffleiste bemisst sich mit 12 mm. Die Größe der freien Grifffläche (57) beträgt somit 10,2 cm². Auf der ersten Stützfolie (44c, vgl. Figur 6 und 7) ist ein 5 mm breites, doppelseitig klebend ausgerüstetes Klebeband mit einer Dicke von 0,08 mm aufgebracht. Dieses Klebeband besteht aus einer transparenten Polyester-Trägerfolie (41) die auf beiden Seiten einen Acrylat-Haftklebstoff-Auftrag trägt. Die erste Seite (42) dieses doppelseitigen Klebebandes ist auf die Stützfolie aufgebracht. Die zweite Seite (48) bildet den adhäsiven Bereich der Stützfolie. Dieser adhäsive Bereich ist mittels eines 0,077 mm dicken Silikonpapiers mit einem Flächengewicht von 92 g/ m²abgedeckt. Ein solches Klebeband ist von der Fa. SwissChem Ag, Widnau - Schweiz zu beziehen. Auf der beschreibbaren Stützfolie ist der Name des Herstellers, der Produktname sowie die offenen Markierungsfelder Datum und Patient aufgebracht. Die Klebkraft des adhäsiven Bereichs der Stützfolie beträgt 0,16 N/ 5 mm gegen Papier (80 g/ m², holzfrei, hochweiß - 3000 Laser Copier der Firma Niceday) Gemessen wurde nach DIN EN 1939 mit dem Unterschied, dass ein Anrollgewicht von 106,7 g verwendet und mittels eines Handrollers aufgetragen wurde, wobei jede Probe insgesamt 4 mal angerollt wurde. Diese Klebkraft wird auch nach mehrmaligem Positionieren auf einer anderen Stelle des Blattes aufrechterhalten. Damit ist die Stützfolie repositionierbar.

Bei der Anwendung dieses Kanülenpflasters wird nun wie zuvor beschrieben im ersten Schritt die Haftklebefläche des Folienverbandes durch Entfernen des silikonisierten Abdeckpapiers freigesetzt. Nun kann der Folienverband wie mit Figuren 8a bis 8c beschrieben appliziert werden, wobei das Ende des Schlitzes (59) in etwa über die Einstichstelle der Kanüle positioniert wird. Die beiden Flügel des Folienverbandes, die mit den Stützfolien (55a, 55b) abgedeckt sind, können parallel zur Kanüle fixiert werden. Mit diesen Bereichen kann jedoch auch die Kanüle weiter festgelegt werden. Mit der bei der Applikation entfernten Stützfolie erhält der Anwender zugleich eine Art Merkzettel an die Hand, mit dem er die Anwendung des zuvor applizierten Kanülenpflasters dokumentieren kann. Hierzu wird lediglich der Abdeckstreifen (49) entfernt und die Stützfolie in die Dokumentationsmappe eingeklebt.

## Patentansprüche

1. Folienverband (10, 30, 40, 60) umfassend einen Polymerfilm (1) und zur leichteren Handhabung des Folienverbandes ein Applikationssystem, wobei das Applikationssystem auf einer ersten Seite des Polymerfilms (1) angeordnet ist und mindestens eine Stützfolie (14, 20, 34, 44c, 64a) umfasst, an der zusätzlich mindestens eine Griffleiste (15, 25, 35, 45, 65) angeformt ist, und wobei die Stützfolie (14, 20, 34, 44c, 64a) eine erste, dem Polymerfilm (1) zugewandte Seite und eine zweite dem Polymerfilm (1) abgewandte Seite aufweist und die zweite Seite der Stützfolie (14, 20, 34, 44c, 64a) mindestens einen adhäsiven Bereich (18, 28, 38, 48, 68) aufweist, und wobei die Stützfolie (14, 20, 34, 44c, 64a) mittels des adhäsiven Bereiches (18, 28, 38, 48, 68) direkt in eine Patienten- oder Dokumentationsmappe klebbar ist.

2. Folienverband (10, 30, 40, 60) nach Anspruch 1, **dadurch gekennzeichnet, dass** der adhäsive Bereich (18, 38, 48, 68) mit einem Abdeckmittel (19, 39, 49, 69), insbesondere mit einem streifenförmigen Abdeckmittel, abgedeckt ist.

3. Folienverband (10, 30, 40, 60) nach mindestens einem der vorangegangenen Ansprüche, **dadurch gekennzeichnet, dass** der Polymerfilm (1) mindestens einen ersten stützfolienfreien Bereich (33, 43, 53, 63, 73) aufweist.

4. Folienverband (10, 30, 40, 60) nach mindestens einem der vorangegangenen Ansprüche **dadurch gekennzeichnet, dass** das Applikationssystem mindestens zwei Stützfolien (44c, 44b, 44a, 64a, 64b) umfasst, die in einer Ebene parallel zum Polymerfilm (1) angeordnet sind.

5. Folienverband (10, 30, 40, 60) nach mindestens einem der vorgenannten Ansprüche **dadurch gekennzeichnet, dass** der Polymerfilm (1) einen ersten (43, 53, 63, 73) und mindestens einen zweiten stützfolienfreien Bereich (43, 53, 63, 73) aufweist, der von dem ersten Bereich getrennt angeordnet ist.

6. Folienverband (10, 30, 40, 60) nach mindestens einem der vorgenannten Ansprüche **dadurch gekennzeichnet, dass** der erste oder der zweite stützfolienfreie Bereich (33, 43) einen Randbereich des Polymerfilms (1) bildet.

7. Folienverband (10, 30, 40, 60) nach mindestens einem der vorangegangenen Ansprüche **dadurch gekennzeichnet, dass** der erste oder der zweite stützfolienfreie Bereich (53, 73) zwischen einer ersten und einer zweiten Stützfolie (44c, 44b, 44a, 64a, 64b) angeordnet ist.

8. Folienverband (10, 30, 40, 60) nach mindestens einem der vorangegangenen Ansprüche, **dadurch gekennzeichnet, dass** mindestens eine Griffleiste (15, 45, 55) den ersten oder den zweiten stützfolienfreien Bereich (13, 53) zumindest abschnittsweise überfängt.

9. Folienverband (10, 30, 40, 60) nach mindestens einem der vorgenannten Ansprüche, **dadurch gekennzeichnet, dass** das Applikationssystem mindestens zwei Stützfolien (44a, 44b, 44c, 64a, 64b) mit jeweils mindestens einer Griffleiste (45, 55a, 55b, 65, 75) umfasst.

10. Folienverband (10, 30, 40, 60) nach mindestens einem der vorgenannten Ansprüche, **dadurch gekennzeichnet, dass** eine der beiden Griffleisten (45, 65) die andere Griffleiste (55, 75) zumindest abschnittsweise überfängt.

11. Folienverband (10, 30, 40, 60) nach mindestens einem der vorgenannten Ansprüche **dadurch gekennzeichnet, dass** die der Stützfolie abgewandte zweite Seite des Polymerfilms (1) mit einem Haftklebemittel, insbesondere vollflächig mit einem Haftklebemittel (2) beschichtet ist und das Haftklebemittel mit einer Abdeckfolie oder einem Abdeckpapier (3) abgedeckt ist.

12. Folienverband (10, 30, 40, 60) nach mindestens einem der vorgenannten Ansprüche **dadurch gekennzeichnet, dass** der Polymerfilm (1) ein Polyurethanfilm, Polyesterurethanfilm oder Polyetherurethanfilm ist.

13. Folienverband (10, 30, 40, 60) nach mindestens einem der vorgenannten Ansprüche **dadurch gekennzeichnet, dass** der Folienverband einen Einschnitt (59) von einem Randbereich zu einem mittleren Bereich aufweist.

14. Verwendung einer Stützfolie (14, 24, 34, 44c, 64a) eines Folienverbandes (10, 30, 40, 60) zur Dokumentation der Anwendung des Folienverbandes.

## Claims

1. Film dressing (10, 30, 40, 60) comprising a polymer film (1) and for easier handling of the film dressing an application system, wherein the application system is arranged on a first side of the polymer film (1) and comprises at least one support film (14, 20, 34, 44c, 64a) onto which in addition at least one gripping strip (15, 25, 35, 45, 65) is formed, and wherein the support film (14, 20, 34, 44c, 64a) has a first side facing the polymer film (1) and a second side facing away from the polymer film (1) and the second side of the support film (14, 20, 34, 44c, 64a) has at least one adhesive region (18, 28, 38, 48, 68) and wherein the support film (14, 20, 34, 44c, 64a) may be adhesively bonded directly into a patient or document folder by means of the adhesive region (18, 28, 38, 48, 68).

2. Film dressing (10, 30, 40, 60) according to Claim 1, **characterized in that** the adhesive region (18, 38, 48, 68) is covered with a covering means (19, 39, 49, 69), in particular with a strip-shaped covering means.

3. Film dressing (10, 30, 40, 60) according to at least one of the preceding claims, **characterized in that** the polymer film (1) has at least one first support-film-free region (33, 43, 53, 63, 73).

4. Film dressing (10, 30, 40, 60) according to at least one of the preceding claims, **characterized in that** the application system comprises at least two support films (44c, 44b, 44a, 64a, 64b) arranged in a plane parallel to the polymer film (1).

5. Film dressing (10, 30, 40, 60) according to at least one of the preceding claims, **characterized in that** the polymer film (1) has a first (43, 53, 63, 73) and at least a second support-film-free region (43, 53, 63, 73) which is disposed separately from the first region.

6. Film dressing (10, 30, 40, 60) according to at least one of the preceding claims, **characterized in that** the first or the second support-film-free region (33, 43) forms an edge region of the polymer film (1).

7. Film dressing (10, 30, 40, 60) according to at least one of the preceding claims, **characterized in that** the first or the second support-film-free region (53, 73) is disposed between a first and a second support film (44c, 44b, 44a, 64a, 64b).

8. Film dressing (10, 30, 40, 60) according to at least one of the preceding claims, **characterized in that** at least one gripping strip (15, 45, 55) overlaps the first or the second support-film-free region (13, 53) at least in sections.

9. Film dressing (10, 30, 40, 60) according to at least one of the preceding claims, **characterized in that** the application system comprises at least two support films (44a, 44b, 44c, 64a, 64b) each having a gripping strip (45, 55a, 55b, 65, 75) .

10. Film dressing (10, 30, 40, 60) according to at least one of the preceding claims, **characterized in that** one of the two gripping strips (45, 65) overlaps the other gripping strip (55, 75) at least in sections.

11. Film dressing (10, 30, 40, 60) according to at least one of the preceding claims, **characterized in that** the second side of the polymer film (1) facing away from the support film has a covering of pressure-sensitive adhesive, in particular an allover covering of pressure-sensitive adhesive (2), and the pressure-sensitive adhesive is covered with a covering film or a covering paper (3).

12. Film dressing (10, 30, 40, 60) according to at least one of the preceding claims, **characterized in that** the polymer film (1) is a polyurethane film, polyester urethane film or polyether urethane film.

13. Film dressing (10, 30, 40, 60) according to at least one of the preceding claims, **characterized in that** the film dressing has a cut (59) from an edge region to a middle region.

14. Use of a support film (14, 24, 34, 44c, 64a) of a film dressing (10, 30, 40, 60) for documenting the use of the film dressing.

## Revendications

1. Pansement film (10, 30, 40, 60) comprenant un film polymère (1) et, pour faciliter la manipulation du pansement film, un système d'application, le système d'application étant disposé sur une première face du film polymère (1) et comprenant au moins une feuille d'appui (14, 20, 34, 44c, 64a), contre laquelle est rapportée au moins une ailette de préhension (15, 25, 35, 45, 65), et la feuille d'appui (14, 20, 34, 44c, 64a) présentant une première face dirigée vers le film polymère (1) et une deuxième face opposée au film polymère (1), et la deuxième face de la feuille d'appui (14, 20, 34, 44c, 64a) comprenant au moins une zone adhésive (18, 28, 38, 48, 68), et la feuille d'appui (14, 20, 34, 44c, 64a) pouvant, à l'aide de la zone adhésive (18, 28, 38, 48, 68), être collée directement dans un dossier de patient ou de documentation.

2. Pansement film (10, 30, 40, 60) selon la revendication 1, **caractérisé en ce que** la zone adhésive (18, 38, 48, 68) est recouverte d'un moyen de recouvrement (19, 39, 49, 59), en particulier d'un moyen de recouvrement en forme de bande.

3. Pansement film (10, 30, 40, 60) selon au moins l'une des revendications précédentes, **caractérisé en ce que** le film polymère (1) comprend au moins une première zone (33, 43, 53, 63, 73) sans feuille d'appui.

4. Pansement film (10, 30, 40, 60) selon au moins l'une des revendications précédentes, **caractérisé en ce que** le système d'application comprend au moins deux feuilles d'appui (44c, 44b, 44a, 64a, 64b), qui sont disposées dans un plan parallèlement au film polymère (1) .

5. Pansement film (10, 30, 40, 60) selon au moins l'une des revendications précédentes, **caractérisé en ce que** le film polymère (1) comprend une première zone (43, 53, 63, 73) et au moins une deuxième zone (43, 53, 63, 73) sans feuille d'appui, qui est disposée séparément de la première zone.

6. Pansement film (10, 30, 40, 60) selon au moins l'une des revendications précédentes, **caractérisé en ce que** la première ou la deuxième zone (33, 43) sans feuille d'appui forme une zone de bordure du film polymère (1) .

7. Pansement film (10, 30, 40, 60) selon au moins l'une des revendications précédentes, **caractérisé en ce que** la première ou la deuxième zone (53, 73) sans feuille d'appui est disposée entre une première et une deuxième feuilles d'appui (44c, 44b, 44a, 64a, 64b).

8. Pansement film (10, 30, 40, 60) selon au moins l'une des revendications précédentes, **caractérisé en ce qu'**au moins une ailette de préhension (15, 45, 55) recouvre au moins par endroits la première ou la deuxième zone (13, 53) sans feuille d'appui.

9. Pansement film (10, 30, 40, 60) selon au moins l'une des revendications précédentes, **caractérisé en ce que** le système d'application comprend au moins deux feuilles d'appui (44a, 44b, 44c, 64a, 64b) ayant chacune au moins une ailette de préhension (45, 55a, 55b, 65, 75).

10. Pansement film (10, 30, 40, 60) selon au moins l'une des revendications précédentes, **caractérisé en ce que** l'une des deux ailettes de préhension (45, 65) recouvre au moins par endroits l'autre ailette de préhension (55, 75).

11. Pansement film (10, 30, 40, 60) selon au moins l'une des revendications précédentes, **caractérisé en ce que** la deuxième face, opposée à la feuille d'appui, du film polymère (1) est revêtue d'un adhésif de contact, en particulier en pleine surface d'un adhésif de contact (2), l'adhésif de contact étant recouvert d'une feuille de recouvrement ou d'un papier de recouvrement (3) .

12. Pansement film (10, 30, 40, 60) selon au moins l'une des revendications précédentes, **caractérisé en ce que** le film polymère (1) est un film de polyuréthanne, un film de polyester-uréthanne ou un film de polyétheruréthanne.

13. Pansement film (10, 30, 40, 60) selon au moins l'une des revendications précédentes, **caractérisé en ce que** le pansement film comprend une encoche (59) d'une zone de bordure à une zone centrale.

14. Utilisation d'une feuille d'appui (14, 24, 34, 44c, 64a) d'un pansement film (10, 30, 40, 60) pour la documentation portant sur l'utilisation du pansement film.
